(19) Europäisches Patentamt
European Patent Office
Office européen des brevets

(11) Veröffentlichungsnummer: **0 245 771**
**B1**

(12) **EUROPÄISCHE PATENTSCHRIFT**

(45) Veröffentlichungstag der Patentschrift: **23.11.89**

(51) Int. Cl.⁴: **C07C 29/15**, B01J 31/28, B01J 31/22, C07C 31/20

(21) Anmeldenummer: **87106591.8**

(22) Anmeldetag: **07.05.87**

(54) **Verfahren zur Herstellung von Ethylenglykol.**

(30) Priorität: **10.05.86 DE 3615835**

(43) Veröffentlichungstag der Anmeldung: **19.11.87 Patentblatt 87/47**

(45) Bekanntmachung des Hinweises auf die Patenterteilung: **23.11.89 Patentblatt 89/47**

(84) Benannte Vertragsstaaten: **BE DE ES FR GB IT NL**

(56) Entgegenhaltungen:
**EP-A- 0 171 632**
**EP-A- 0 215 378**

**JOURNAL OF THE CHEMICAL SOCIETY CHEMICAL COMMUNICATIONS, Nr. 12, 1986, Seiten 900-901; H.W. BOSCH et al.: "The role of rhodium porphyrins in the photoassisted formation of famaldehyde and methanol from hydrogen and carbon monoxide"**

(73) Patentinhaber: **BASF Aktiengesellschaft, Carl-Bosch-Strasse 38, D-6700 Ludwigshafen(DE)**

(72) Erfinder: **Maerkl, Robert, Dr., Schulstrasse 17, D-6701 Fussgoenheim(DE)**
Erfinder: **Harder, Wolfgang, Dr., Bergwaldstrasse 16, D-6940 Weinheim(DE)**

**EP 0 245 771 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein verbessertes Verfahren zur Herstellung von Ethylenglykol aus Kohlenmonoxid und Wasserstoff in homogener flüssiger Phase unter erhöhtem Druck und bei erhöhter Temperatur in Gegenwart Rhodium enthaltender Katalysatoren sowie in Gegenwart organischen Stickstoff enthaltender Promotoren.

Diese Direktsynthese von Ethylenglykol aus Kohlenmonoxid und Wasserstoff, bei der als Nebenprodukt u.a. Methanol und Ethanol anfallen, ist, abgesehen von der erfindungsgemäßen Verbesserung, allgemein bekannt, z.B. aus der EP 0 012 924 oder der DE-OS 26 43 897. Als Promotoren werden dort stickstoffhaltige Lewis-Basen, z.B. Pyridine wie 2-Hydroxypyridin oder N-alkylsubstituierte Morpholine verwendet.

Die Abtrennung des Ethylenglykols aus dem rohen Reaktionsgemisch wird in der Regel durch wäßrige Extraktion vorgenommen. Dabei zeigen viele der gemäß dem Stand der Technik bevorzugten Promotoren den Nachteil, in wäßriger Lösung löslich zu sein, so daß bei der Aufarbeitung Probleme auftreten. Weiterhin läßt die Stabilität der aus Katalysatormetall und Promotor gebildeten Komplexe, insbesondere bei niedrigen Drucken zu wünschen übrig.

Der Erfindung lag daher die Aufgabe zugrunde, neuartige Promotoren für die Direktsynthese von Ethylenglykol bereitzustellen, die den Rhodium enthaltenden homogenen Katalysator stabilisieren und zugleich aktivieren und in Wasser weitgehend unlöslich sind, so daß keine Aufarbeitungsprobleme auftreten. Durch die Stabilisierung des Katalysatorsystems sollte es möglich sein, dieses für weitere Umsetzungen wiederzuverwenden, ohne starke Produktivitätseinbußen hinnehmen zu müssen.

Demgemäß wurde ein Verfahren zur Herstellung von Ethylenglykol aus Kohlenmonoxid und Wasserstoff in homogener flüssiger Phase unter erhöhtem Druck und bei erhöhter Temperatur in Gegenwart Rhodium enthaltender Katalysatoren sowie in Gegenwart organischer, Stickstoff enthaltender Promotoren gefunden, das dadurch gekennzeichnet ist, daß man als Promotoren Porphyrine verwendet.

Als Porphyrine kommen z.B. Verbindungen der allgemeinen Formel I

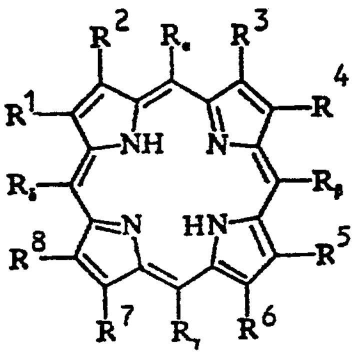

I

in Betracht, in der die Reste $R_\alpha$ bis $R_\delta$ Wasserstoff, Alkyl-, Cycloalkyl-, Aryl-, Aralkyl- oder heterocyclische Reste bedeuten, $R^1$ bis $R^8$ für Wasserstoff, Alkyl-, Alkenyl-, Cycloalkyl- Aryl-, Aralkylreste oder heterocyklische Reste stehen und je zwei benachbarte Reste einer Pyrroleinheit auch miteinander zu einem 5- oder gegebenenfalls aromatischen 6-Ring verknüpft sein können. Die organische Reste können noch unter den Reaktionsbedingungen inerte Substituenten tragen, wobei man aus verfahrenstechnischen Gründen solche Substituenten wählen wird, die wenig hydrophil sind, z.B. $C_1$-$C_4$-Alkoxyreste.

Als Alkylreste können vorteilhaft solche mit 1 bis 20, vorzugsweise 1 bis 10, insbesondere 1 bis 5 Kohlenstoffatomen vorliegen, z.B. die Methyl-, Ethyl-, Propyl-, Isopropyl-, Butyl-, Isobutyl-, Pentyl oder Isopentylgruppe. Alkenylreste sind z.B. Vinyl- oder Allylreste, Cycloalkylreste sind z.B. Cyclopentyl- oder Cyclohexylreste. Als Arylrest wäre beispielsweise der Phenylrest zu nennen, der durch einen oder mehrere Alkylreste, z.B. $C_1$- bis $C_4$-Alkylreste substituiert sein kann. Aralkylreste sind insbesondere solche mit 7 bis 12 C-Atomen, z.B. der Benzylrest. Als hetrocyclische Reste kommen vor allem Stickstoff enthaltende 5- oder 6-gliedrige Ringe in Betracht, z.B. der Pyridylrest.

Zwecks einfacher Aufarbeitung und Wiederverwendung der porphyrinstabilisierten Katalysatoren für eine erneute Umsetzung, werden vorzugsweise weitgehend wasserunlösliche Porphyrine verwendet. Wegen ihrer leichten Zugänglichkeit sind solche Porphyrine bevorzugt, in denen $R^1$ bis $R^8$ gleiche oder verschiedene Alkylreste mit 1 bis 4 Kohlenstoffatomen, insbesondere Methyl- oder Ethylreste darstellen und $R_\alpha$ bis $R_\delta$ für Wasserstoff stehen oder in denen $R^1$ bis $R^8$ Wasserstoff bedeuten und $R_\alpha$ bis $R_\delta$ Phenyl-, Pyridyl- oder Tolylreste sind. Beispielsweise seien Octaethylporphyrin, Tetramethyltetraethylporphyrin, α,β, γ, δ-Tetraphenylporphyrin oder α, β, γ,δ-Tetrapyridylporphyrin genannt.

Die Herstellung der Porphyrine kann nach dafür bekannten Methoden, z.B. wie in B.D. Berezin: Coordination Compounds of Porphyrins and Phthalocyanines John Wiley & Sons, Kap. 2, 1981 oder Albert Gossauer: Chemie der Pyrrole, Springer-Verlag, 1974, S. 117/118, beschrieben, erfolgen.

Die nach folgender Reaktionsgleichung ablaufende Direktsynthese von Ethylenglykol

$$2\ CO + 3\ H_2 \xrightarrow{Kat.} HO{-}CH_2{-}CH_2{-}OH$$

erfolgt, abgesehen von der erfindungsgemäßen Verwendung des Porphyrinpromotors in an sich bekannter Weise, z.B. wie in der älteren EP-A 215 378 beschrieben.

Als Katalysatoren verwendet man entweder Rhodium allein oder zusammen mit anderen carbonylbildenden Metallen wie Ruthenium, Palladium, Platin, Iridium, Eisen, Nickel und besonders Cobalt, wobei der Anteil des Rhodiums mindestens 5 Mol% betragen sollte.

Da sich die aktiven Carbonylkomplexe während der Reaktion von selber bilden, kann man die Katalysatoren in metallischer Form oder vorzugsweise in Form ihrer Salze oder Komplexverbindungen wie den Carbonylkomplexen oder Komplexen mit beispielsweise Acetylaceton oder Porphyrin einsetzen.

Besonders bevorzugt werden die in der DE-OS 34 27 138 beschriebenen Mischkatalysatoren oder Katalysatorgemische mit Rhodium und Cobalt als aktiven Metallen verwendet, in denen das Molverhältnis von Rhodium zu Cobalt etwa 20:1 bis 60:1, insbesondere 25:1 bis 30:1, beträgt.

Die Rhodiumkonzentration bzw. die Gesamtkonzentration der carbonylbildenden Metalle im Reaktionsmedium kann im Prinzip beliebig sein, da sie im wesentlichen nur einen Einfluß auf die Reaktionsgeschwindigkeit und damit auf die Raum-Zeit-Ausbeute hat. Befriedigende Raum-Zeit-Ausbeuten werden in der Regel im Konzentrationsbereich von 0,01 - 0,5 Gew.-% Metall erzielt; höhere Konzentrationen bringen keine nennenswerten wirtschaftlichen Vorteile mehr, und bei geringeren Konzentrationen - etwa bis herab zu 0,005 Gew.-% verlangsamt sich die Reaktion entsprechend.

Für den Gesamtdruck empfiehlt sich ein Bereich von 300 - 3000, vorzugsweise 600 - 2000 bar, wobei der Partialdruck des Kohlenmonoxids vorzugsweise zwischen 20 und 80 %, besondere zwischen 30 und 60 % des Gesamtdruckes liegt.

Gute Ergebnisse erzielt man bei Temperaturen von 180 - 280°C, wobei der Bereich von 200 - 240°C im allgemeinen zu bevorzugen ist.

Als Lösungsmittel für die Glykolsynthese eignen sich die für die Direktsynthese bekannten organischen Lösungsmitteln wie n- und iso-Alkanole. Von diesen Lösungsmitteln sind $C_2$-$C_{20}$n-Alkanole, insbesondere $C_2$-$C_8$-Alkanole, vorzugsweise die in Wasser wenig löslichen $C_5$-$C_8$-Alkanole besonders gut geeignet. Der Zusatz von Verbindungen wie Tetraalkylharnstoffen, Lactonen, sowie N-Aryl- und N-Alkylpyrrolidonen und -imidazolidonen, besonders N-Methylpyrrolid-2-on, 1,5-Dimethylpyrrolid-2-on und 1,3-Dimethylimidazolid-2-on kann sich vorteilhaft auf die Reaktion auswirken. Die Verwendung von Lösungsmitteln mit geringer Wasserlöslichkeit gestattet es, die gebildeten Syntheseprodukte Ethylenglykol, Methanol und Ethanol durch einfache Extraktion mit Wasser aus dem Reaktionsgemisch zu gewinnen. Der weitgehend wasserunlösliche durch Porphyrin stabilisierte Katalysator verbleibt in der organischen Phase, die sich unmittelbar für eine weitere Glykolsynthese wiederverwenden läßt.

Die Menge des Porphyrins liegt vorzugsweise bei 0,1 bis 10 mol, insbesondere 0,5 bis 5 mol, pro Mol der Zentralmetalle. Durch Zugabe der Porphyrinpromotoren entstehen Komplexe, bestehend aus Zentralatom, z.B. Rhodium oder Cobalt, das ein oder mehrere Porphyrine als Ligand trägt. Anstatt diese Komplexe in situ herzustellen, ist es auch möglich, diese vorab zu synthetisieren, z.B. nach der von H. Ogoshi et al in J. of Amer. Chem. Soc. 97, 6461-6465, 1975 beschriebenen Methode. Vorteilhaft bestehen die getrennt hergestellten Komplexe aus einem oder mehreren Rhodiumatomen und im allgemeinen 0,5 bis 1 Porphyrin-Liganden je Zentralmetall.

Die Durchführung der Reaktion kann diskontinuierlich oder kontinuierlich nach den dafür üblichen Techniken erfolgen. Die Aufarbeitung der Reaktionsgemische kann man destillativ oder vorteilhaft durch Extraktion mit Wasser und anschließende Fraktionierung der Extraktphase vornehmen. Die nach wäßriger Extraktion zurückbleibende katalysatorhaltige organische Phase läßt sich für einen weiteren Reaktionsansatz wiederverwenden, so daß sich diese Arbeitsweise verfahrenstechnisch besonders gut für den kontinuierlichen Betrieb eignet.

Beispiel 1

Eine Lösung aus 1,0 g $Rh(CO)_2$ ($CH_3COCH_2COCH_3$) = 0,4 g Rh, 0,026 g $Co_2(CO)_8$ = 0,009 g Co und 0,11 g α,β,γ,δ -Tetraphenylporphyrin in 179,0 g Hexanol-1 und 19,8 g 1,5-Dimethylpyrrolidon-2 wurde 5 Stunden bei 230°C und einem Gesamtdruck von 1500 bar mit einem äquimolaren $CO/H_2$-Gemisch zur Reaktion gebracht. Die gaschromatographisch ermittelten Ausbeuten betrugen:

| | |
|---|---|
| Ethylenglykol | 71,0 g |
| Methanol | 13,5 g |
| Ethanol | 2,7 g |

## Patentansprüche

1. Verfahren zur Herstellung von Ethylenglykol aus Kohlenmonoxid und Wasserstoff in homogener flüssiger Phase unter erhöhtem Druck und bei erhöhter Temperatur in Gegenwart Rhodium enthaltender Katalysatoren sowie in Gegenwart organischer, Stickstoff enthaltender Promotoren, dadurch gekennzeichnet, daß man als Promotoren Porphyrine verwendet.

2. Verfahren nach Anspruch 1, dadurch gekennzeichnet, daß man Porphyrine der allgemeinen Formel I

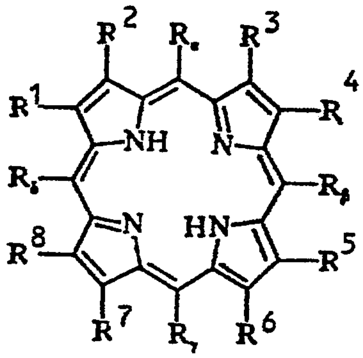

I

verwendet, in der die Reste $R_\alpha$ bis $R_\delta$ Wasserstoff, Alkyl-, Cycloalkyl-, Aryl-, Aralkyl- oder heterocyclische Reste bedeuten, $R^1$ bis $R^8$ für Wasserstoff, Alyl-, Alkenyl-, Cycloalkyl- Aryl-, Aralkylreste oder heterocyklische Reste stehen und je zwei benachbarte Reste einer Pyrroleinheit auch miteinander zu einem 5- oder gegebenenfalls aromatischen 6-Ring verknüpft sein können.

3. Verfahren nach den Ansprüchen 1 und 2, dadurch gekennzeichnet, daß im Reaktionsgemisch 0,1 bis 10 mol Porphyrin pro Mol Metallatom vorhanden sind.

4. Verfahren nach den Ansprüchen 1 bis 3, dadurch gekennzeichnet, daß man als Katalysator einen Rhodium/Cobalt-Mischkomplex oder eine Mischung aus einem Rhodium- und einem Cobaltkomplex verwendet, wobei das Molverhältnis von Rhodium zu Cobalt etwa 20:1 bis 60:1 beträgt.

## Claims

1. A process for preparing ethylene glycol from carbon monoxide and hydrogen in a homogeneous liquid phase under superatmospheric pressure and at elevated temperatures in the presence of a rhodium-containing catalyst and in the presence of an organic nitrogen-containing promotor, which comprises using a porphyrin as promotor.

2. A process as claimed in claim 1, wherein the porphyrin used has the formula I

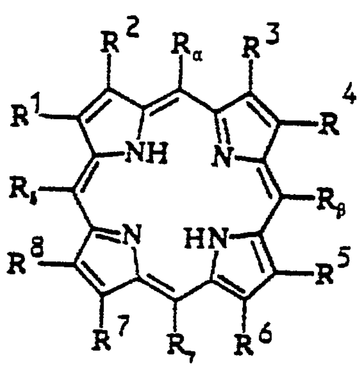

I

where $R_\alpha$, $R_\beta$, $R_\gamma$ and $R_\delta$ are each hydrogen or alkyl, cycloalkyl, aryl, aralkyl or heterocyclic radicals, $R^1$, $R^2$, $R^3$, $R^4$, $R^5$, $R^6$, $R^7$ and $R^8$ are each hydrogen or alkyl, alkenyl, cycloalkyl, aryl, aralkyl or heterocyclic radicals, and pairs of adjacent radicals of a pyrrole unit can also be linked to one another to form a 5- or an aromatic or nonaromatic 6-ring.

3. A process as claimed in either of claims 1 and 2, wherein from 0.1 to 10 moles of porphyrin per mole of metal atom are present in the reaction mixture.

4. A process as claimed in any of claims 1 to 3, wherein the catalyst used in a rhodium/cobalt mixed complex or a mixture of a rhodium and a cobalt complex, with a molar ratio of rhodium: cobalt of from about 20:1 to 60:1.

## Revendications

1. Procédé de préparation d'éthylèneglycol à partir de monoxyde de carbone et d'hydrogène en phase liquide homogène sous pression élevée et à température élevée en présence de catalyseurs contenant du rhodium ainsi qu'en présence de promoteurs organiques contenant de l'azote, caractérisé en ce qu'on utilise comme promoteurs des porphyrines.

2. Procédé selon la revendication 1, caractérisé en ce qu'on utilise des porphyrines de formule générale I

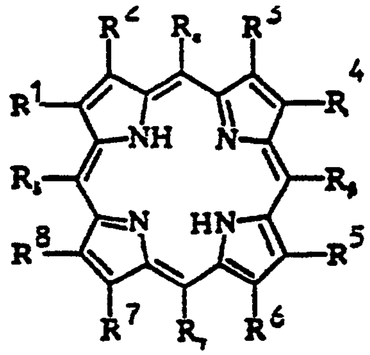

I

dans laquelle les restes $R_\alpha$ à $R_\delta$ sont des atomes d'hydrogène ou des restes alkyle, cycloalkyle, aryle, aralkyle ou hétérocycliques, $R^1$ à $R^8$ sont des atomes d'hydrogène ou des restes alkyle, alcényle, cycloalkyle, aryle, aralkyle ou hétérocycliques et deux restes voisins d'une unité pyrrole peuvent être rattachés l'un à l'autre en formant un noyau à 5 chaînons ou un noyau à 6 chaînons éventuellement aromatique.

3. Procédé selon la revendication 1 ou 2, caractérisé en ce que, dans le mélange réactionnel sont présentes 0,1 à 10 moles de porphyrine par mole d'atome métallique.

4. Procédé selon les revendications 1 à 3, caractérisé en ce qu'on utilise comme catalyseur un complexe mixte rhodium/cobalt ou un mélange d'un complexe de cobalt et d'un complexe de rhodium, le rapport molaire du rhodium au cobalt s'élevant à environ 20:1 à 60:1.